(19) Europäisches Patentamt
European Patent Office
Office européen des brevets

(11) **EP 2 799 112 A1**

(12) **EUROPEAN PATENT APPLICATION**
published in accordance with Art. 153(4) EPC

(43) Date of publication:
**05.11.2014 Bulletin 2014/45**

(51) Int Cl.:
*A61N 7/00* (2006.01)     *A61B 18/04* (2006.01)
*A61B 8/08* (2006.01)

(21) Application number: **11878766.2**

(22) Date of filing: **29.12.2011**

(86) International application number:
**PCT/KR2011/010338**

(87) International publication number:
**WO 2013/100235 (04.07.2013 Gazette 2013/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(71) Applicant: **Alpinion Medical Systems Co., Ltd.**
**Hwaseong-si, Gyeonggi-do 445-380 (KR)**

(72) Inventors:
• **KIM, Dae Seung**
  **Seoul 157-882 (KR)**

• **SON, Keonho**
  **Seongnam-si**
  **Gyeonggi-do 463-970 (KR)**

(74) Representative: **Thorniley, Peter et al**
**Kilburn & Strode LLP**
**20 Red Lion Street**
**London WC1R 4PJ (GB)**

(54) **APPARATUS AND METHOD FOR FORMING BEAM FOR ENHANCING BEAM-FORMING RESOLUTION**

(57)     The present disclosure in one or more embodiments provides a beamforming apparatus and method for enhancing beamforming resolution. The beamforming method comprises quasi-randomly selecting a driving frequency for each channel from two or more candidate frequencies; setting a phase for each channel based on a wavelength of the driving frequency for each channel and a distance between a physical position of each channel and a focal point; and generating the ultrasound waves at the multiple channels, according to the driving frequency and phase selected for each channel.

*FIG. 2*

**Description**

[Technical Field]

The present disclosure in one or more embodiments relates to an apparatus and a method for forming a beam for enhancing beamforming resolution. More particularly, the present disclosure relates to a beamforming apparatus and method for reducing a beamforming error and a grating lobe by enhancing focal resolution of a beam formed in a medical device using, for example, HIFU (High-Intensity Focused Ultrasound) technology.

[Background]

The statements in this section merely provide background information related to the present disclosure and may not constitute prior art.

An ultrasound diagnosis and treatment of the human body has been under active research and development since it obviates the need for an incision of the human body and thus leaves neither a surgical scar nor risk of secondary infection. Exemplary applications of the ultrasonic wave or ultrasound in medical area include diagnosis such as fetal diagnosis or cancer diagnosis and treatment such as a lipectomy or destruction of a cancer cells or malignant tumors.

A HIFU treatment is a medical procedure such as burning cancer cells by intensively irradiating cancer tissue with ultrasound waves. Although HIFU was originally developed for treating prostate cancer, applications thereof have gradually extended to non-solid tumors such as brain cancer, uterine myoma and arrhythmia beyond a solid cancer such as liver cancer, breast cancer, pancreatic cancer and the like. Especially, HIFU presents excellent performance in the treatment of liver cancer and the treatment of pancreatic cancer, a surgical operation against which is impossible.

HIFU treatment has various aspects such as a thermal effect, a cavitation effect, a mechanical effect, destruction of capillaries near a tumor and an immunity effect. Herein, the thermal effect serves to cause blood coagulation and necrose tumor cells by use of heat above 65 degrees. The cavitation effect denatures the structure of protein and thereby destroys DNA of a tumor by pressurizing cells. In addition, the mechanical effect severs a chemical link between cancer cells. The destruction of capillaries adjacent to a tumor is to prevent the tumor from proliferating by destroying the nearby capillaries so as not to supply nutrition to the tumor as well as a lesion to be treated. The immunity effect relates to increasing the level of immunity as with increasing lymphocyte by recognizing a tumor cell destroyed after treatment as an antigen. Among these effects, HIFU based treatment using the thermal effect is one of the most popular kinds.

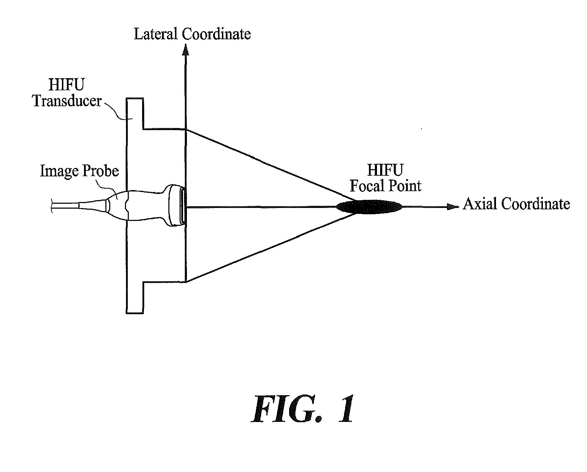

FIG. 1 is a diagram of an example of a HIFU transducer and a beam generated from the HIFU transducer.

Ultrasonic sound energy irradiated from the HIFU transducer is focused on one point, thereby forming a focal point. However, in the HIFU transducer according to the related art, the size of a beam focus formed on a lateral coordinate is 1 to 3 mm, whereas a beam focus formed on an axial coordinate is as large as 5 to 10 mm. This possibly ends up damaging untargeted surrounding organs in addition to cancer cells or tumors during HIFU treatment of a patient because of poor axial resolution in spite of precise treatment available in the direction of the lateral coordinate.

[Disclosure]

[Technical Problem]

Therefore, the present disclosure has been made in an effort to reduce a beamforming error and a grating lobe by increasing resolution of a focal point formed by an ultrasound wave in a medical device using HIFU or other technologies.

[Summary]

In accordance with some embodiments of the present disclosure, a beamforming apparatus is provided for forming a beam focus by generating ultrasound waves at multiple channels. The beamforming apparatus comprises a frequency selector, a phase selector and a beamforming signal generator. The frequency selector is configured to quasi-randomly select a driving frequency for each channel from two or more candidate frequencies. The phase selector is configured to set a phase for each channel based on a wavelength of the driving frequency for each channel and a distance between a physical position of each channel and a focal point. The beamforming signal generator is configured to control generation of the ultrasound waves at the multiple channels, according to the driving frequency and the phase selected for each channel.

The candidate frequencies may be frequencies exhibiting a response characteristic of 3 dB or more.

The driving frequency for each channel may be selected randomly and differently from that of an adjacent channel.

The driving frequency for each channel may be randomly selected so that selected driving frequencies have a uniform distribution.

The driving frequency for each channel may be randomly selected, so that the driving frequency for each channel is different from that of an adjacent channel and selected driving frequencies have uniform distribution.

The beamforming apparatus may further comprise an amplitude selector configured to set an amplitude for each channel, wherein the beamforming signal generator generates the ultrasound waves according to the driving frequency, the phase and the amplitude selected for each channel.

The amplitude for each channel may be differently set according to a distance from a center of a transducer of the beamforming apparatus to each channel.

The amplitude for each channel may be selected randomly and differently from that of an adjacent channel.

The amplitude for each channel may be set such that amplitudes are distributed by equal ratio with respect to individual channels.

In accordance with some embodiments of the present disclosure, a beamforming method performed by a beamforming apparatus is provided for forming a beam focus by generating ultrasound waves at multiple channels. The beamforming method comprises quasi-randomly selecting a driving frequency for each channel from two or more candidate frequencies; setting a phase for each channel based on a wavelength of the driving frequency for each channel and a distance between a physical position of each channel and a focal point; and generating the ultrasound waves at the multiple channels, according to the driving frequency and the phase selected for each channel.

The driving frequency for each channel may be selected randomly and differently from that of an adjacent channel.

The driving frequency for each channel is randomly selected so that the driving frequency for each channel is different from that of an adjacent channel and selected driving frequencies have uniform distribution.

The beamforming method may further comprise differently setting an amplitude for each channel according to a distance from a center of a transducer of the beamforming apparatus to each channel. The ultrasound waves are generated according to the driving frequency, the phase and the amplitude selected for each channel.

[Advantageous Effects]

According to the present disclosure as described above, a beamforming error can be reduced by increasing resolution of a focal point formed by an ultrasound wave generated in a medical device using HIFU or other technologies.

In addition, according to the present disclosure as described above, a grating lobe can be reduced.

[Description of Drawings]

FIG. 1 is a diagram of an example of a HIFU transducer and a beam generated from the HIFU transducer.

FIG. 2 is a block diagram of a beamforming apparatus according to at least one embodiment of the present disclosure.

FIG. 3 is an exemplary diagram of types of a HIFU transducer.

FIG. 4 is an enlarged diagram of some channel elements in a rectangular transducer array.

FIG. 5 is an exemplary diagram of a frequency response of each channel in a HIFU transducer.

FIG. 6 is an exemplary diagram of distances between a focal point and two channels.

FIG. 7 is an exemplary diagram of a transducer divided into multiple regions from a center point of the transducer.

FIG. 8 is an exemplary diagram of a conventional waveform (dotted) together with the patterns of waveform at various depths of irradiation with candidate frequencies set at an interval of 0.1 MHz within a range of 0.8 MHz to 1.2 MHz and after generating signals randomly over 92 channels for each driving frequency from the candidate frequencies.

FIG. 9 is a flowchart of an example beamforming method according to at least one embodiment of the present disclosure.

[Detailed Description]

Hereinafter, at least one embodiment of the present disclosure will be described in detail with reference to the accompanying drawings. In the following description, like reference numerals designate like elements although the elements are shown in different drawings. Further, in the following description of the at least one embodiment, a detailed description of known functions and configurations incorporated herein will be omitted for the purpose of clarity and brevity.

Additionally, in describing the components of the present disclosure, terms like first, second, A, B, (a), and (b) are used. These are solely for the purpose of differentiating one component from another, and one of ordinary skill would understand that the terms are not to imply or suggest the substances, order or sequence of the components. If a component is described as 'connected', 'coupled', or 'linked' to another component, one of ordinary skill in the art would understand that the components are not necessarily directly 'connected', 'coupled', or 'linked' but also are indirectly 'connected', 'coupled', or 'linked' via a third component.

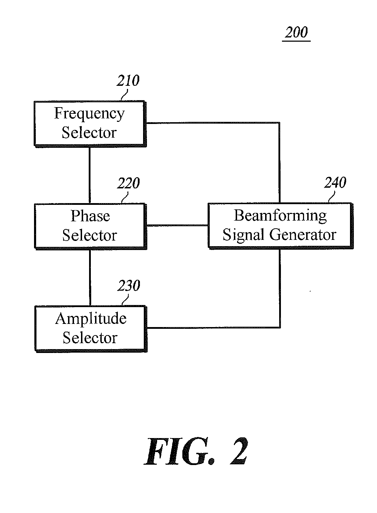

FIG. 2 is a block diagram of a beamforming apparatus according to at least one embodiment of the present disclosure.

As illustrated in FIG. 2, a beamforming apparatus 200 according to at least one embodiment of the present disclosure may include, as a HIFU transducer for example, a frequency selector 210, a phase selector 220, an amplitude selector 230 and a beamforming signal generator 240. Herein, components included in the beamforming apparatus 200 are merely for exemplary description of the technological idea of at least one embodiment of the present disclosure. Therefore, those skilled in the art will appreciate that various modifications and substitutions for the components included in the beamforming apparatus 200 are possible, without departing from the essential characteristics of at least one embodiment of the present disclosure.

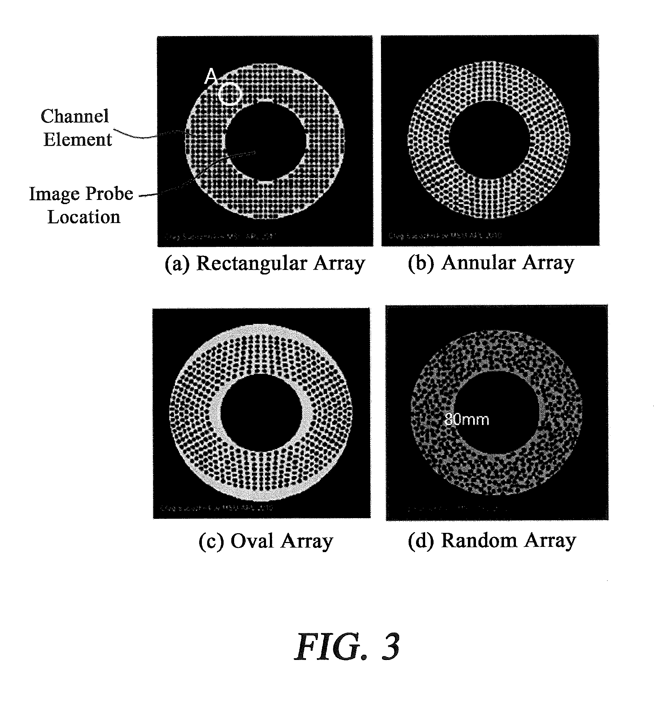

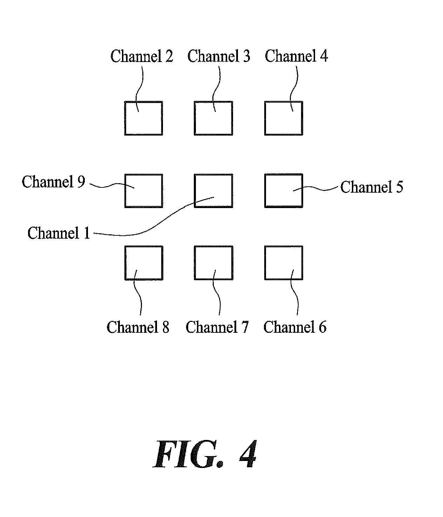

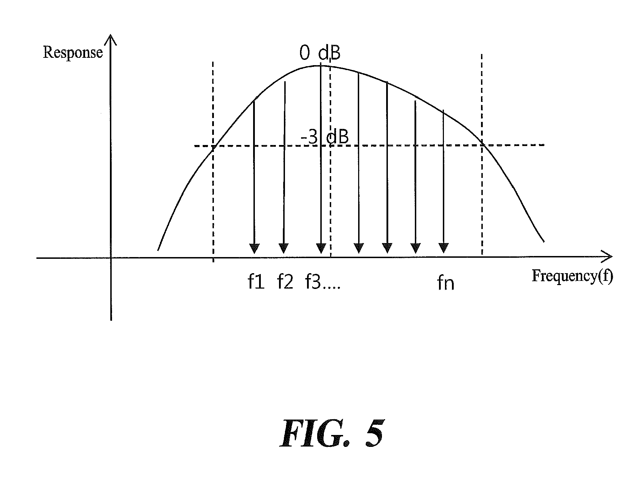

FIG. 3 is an exemplary diagram of types of a HIFU transducer. FIG. 4 is an enlarged diagram of area "A" of channel elements in a rectangular array of FIG. 3 and FIG. 5 is an exemplary diagram of a frequency response of each channel in a HIFU transducer.

The HIFU transducer generates ultrasound waves from its plurality of channels and each of the channels includes an element for irradiating an ultrasound beam.

As illustrated in FIG. 3, the HIFU transducer may have various types including (a) rectangular array, (b) annular array, (c) oval array, and (d) random array. Each array includes a plurality of channel elements and the HIFU transducer has various types according to an arranged form of the channel elements.

The frequency selector 210 selects two or more candidate frequencies from the frequencies exhibiting a response characteristic of 3 dB or more in the HIFU transducer and quasi-randomly selects, from the two or more candidate frequencies, a driving frequency per ultrasound beam for each channel. The meaning of quasi-random selection of a driving frequency is that the driving frequency may be randomly selected but additional conditions may be applied.

FIG. 5 illustrates selecting frequencies of 3 dB or more for the candidate frequencies in consideration of characteristics of individual frequencies of an ultrasound wave transmitted via the human body as a medium. Herein, frequencies selected as the candidate frequencies are f1, f2, ..., fn. The candidate frequencies are at least two frequencies.

The frequency selector 210 may randomly select the driving frequency for each channel from the candidate frequencies in such a manner that adjacent channels have different frequencies. As illustrated in FIG. 4, if f1 is randomly selected as a driving frequency of an element of channel 1, frequencies other than f1 are selected as driving frequencies of channel 2, channel 3, channel 4,..., channel 9. Herein, while channel 2 to channel 9 have been exemplified as adjacent channels, channel 3, channel 5, channel 7, and channel 9 may be set as adjacent channels of channel 1. In addition, adjacent channel elements may be defined in various manners.

Moreover, the frequency selector 210 may be configured to select driving frequencies for individual channels so that the driving frequencies have a uniform distribution. For example, when driving frequencies are f1, f2, f3, and f4, if the number of channel elements is 240, the number of channel elements allocated to generate ultrasound waves at each driving frequency is 60.

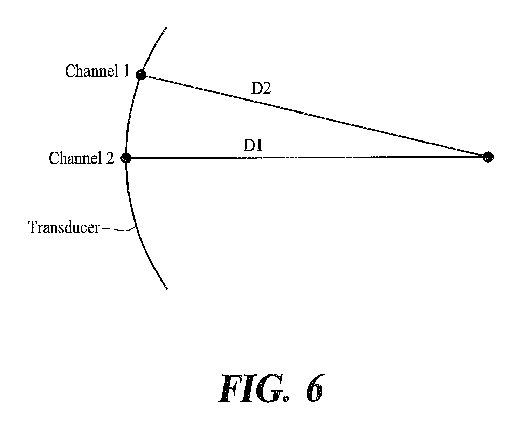

FIG. 6 is a diagram of an example of distances between a focal point and two channels.

The phase selector 220 sets a phase in consideration of the wavelength of a driving frequency selected with respect to each ultrasound wave for each channel and the distance between the physical position of a channel element and the focal point.

As illustrated in FIG. 6, if the distance between channel 1 and the focal point is D1 and the distance between channel 2 and the focal point is D2, phases of ultrasound waves generated in the channels are set such that an ultrasound wave of channel 1 and an ultrasound wave of channel 2 have a phase difference of $2\pi(D2 - D1)/\lambda_2$ where $\lambda_2$ is the wavelength of the ultrasound wave of channel 2.

The beamforming signal generator 240 may generate an ultrasound wave for each channel according to the driving frequency and the phase selected for each channel.

The amplitude selector 230 sets an amplitude for each channel.

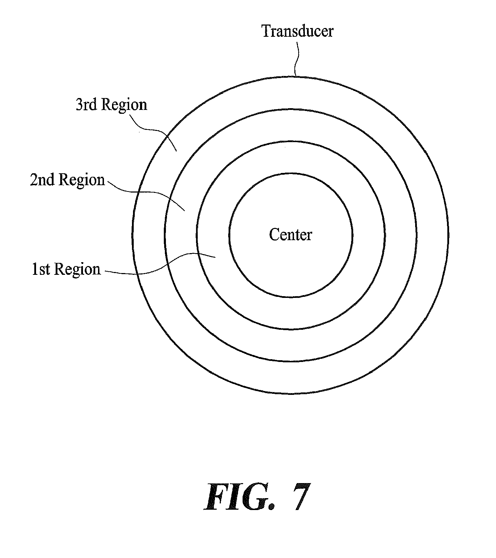

FIG. 7 is a diagram of an example of a transducer divided into multiple regions based on a center point of the transducer.

The amplitude selector 230 may use a method for differently setting the amplitude for each channel according to a distance between the center of the transducer (i.e. the center of position of an image probe) and each channel, as illustrated in FIG. 7. For example, the amplitude may be set to increase as a channel nears the center of the transducer. In other words, the following relationship may be satisfied: amplitude of an ultrasound wave of a channel in region 1 > amplitude of an ultrasound wave of a channel in region 2 > amplitude of an ultrasound wave of a channel in region 3. Such a method for setting the amplitude for each channel is not limited to the exemplary method of the present embodiment and various other methods may be used.

The amplitude selector 230 may randomly select the amplitude for each channel within a prescribed range. The amplitude selector 230 may randomly select the amplitude for each channel within a prescribed range with the proviso that the amplitude for each channel is different from that of an adjacent channel.

In addition, the amplitude for each channel may be set such that amplitudes are distributed by equal ratio with respect to channels.

The beamforming signal generator 240 may control generation of the ultrasound wave at each channel , according to the driving frequency, the phase and the amplitude selected for each channel.

A beam-formed ultrasound signal generated from the control of the beamforming signal generator 240 may be expressed as Equation 1.

$$p = \sum_{n=1}^{n} p_n A_n \exp(j\Phi_n)$$

Equation 1

where $p_n$ is a sound field of an n-th channel, An is an amplitude of an n-th channel,
$\Phi_n$ $(= 2\pi(D_n - D_1)/\lambda_n)$ is a phase difference between an n-th channel and the first channel,
$D_n$ is a distance between an n-th channel and a focal point, D1 is a distance between the first channel and a focal point, and,
$\lambda_n$ is a wavelength of an ultrasound signal of an n-th channel.

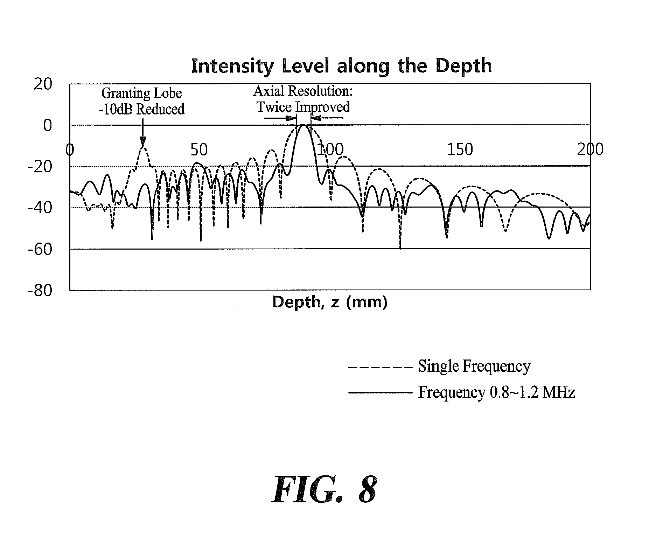

FIG. 8 is an exemplary diagram of a conventional waveform (dotted) together with the patterns of waveform at various depths of irradiation with candidate frequencies set at an interval of 0.1 MHz within a range of 0.8 MHz to 1.2 MHz and after generating signals randomly over 92 channels for each driving frequency from the candidate frequencies.

As illustrated in FIG. 8, as compared with a waveform (a dotted waveform) according to a conventional method generating an ultrasound wave by using a single frequency, it is appreciated that a waveform (a solid waveform) according to a method of at least one embodiment of the present disclosure reduces a grating lobe by -10 dB and improves the axial resolution.

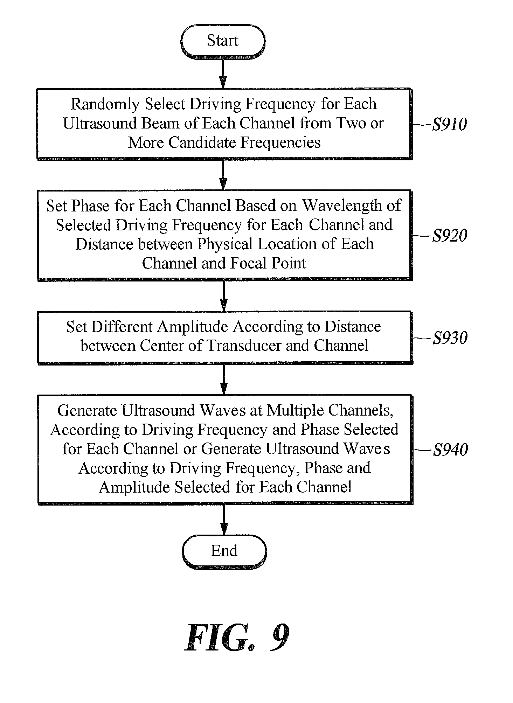

FIG. 9 is a flowchart of a beamforming method according to at least one embodiment of the present disclosure.

As illustrated in FIG. 9, a beamforming method according to at least one embodiment of the present disclosure includes a process for selecting a frequency (S910), a process for selecting a phase (S920), a process for selecting an amplitude (S930), and a process for generating a beamforming signal (S940). The processes included in the beamforming method are merely for exemplary description of the technological idea of at least one embodiment of the present invention. Therefore, those skilled in the art will appreciated that various modifications and substitutions for the processes are possible, without departing from the essential characteristics of at least one embodiment of the present disclosure.

In selecting the frequency (S910), a driving frequency is randomly selected with respect to an ultrasound beam for each channel among two or more candidate frequencies.

The selection of the phase (S920) is to set a phase in consideration of the wavelength of the driving frequency selected with respect to each ultrasound wave for each channel and of the distance between a physical position of a channel element and a focal point.

In selection of the amplitude (S930), a different amplitude is set according to a distance between the center of a transducer and a channel.

The generation of the beamforming signal (S940) is to generate an ultrasound wave according to the driving frequency and the phase selected per channel or generate an ultrasound wave according to the driving frequency, the phase, and the amplitude selected per channel.

The selection of the frequency (S910), the selection of the phase (S920), the selection of the amplitude (S930), and the generation of the beamforming signal (S940) correspond to the frequency selector 210, the phase selector 220, the amplitude selector 230, and the beamforming signal generator 240, respectively, and therefore detailed descriptions thereof are not given.

Although exemplary embodiments of the present disclosure have been described for illustrative purposes, those skilled in the art will appreciate that various modifications, additions and substitutions are possible, without departing from the essential characteristics of the disclosure. Therefore, exemplary embodiments of the present disclosure have been described for the sake of brevity and clarity. Accordingly, one of ordinary skill would understand the scope of the disclosure is not limited by the explicitly described above embodiments but by the claims and equivalents thereof.

[Industrial Applicability]

As described above, the present disclosure is highly useful for applications in a medical device using HIFU or other technologies for reducing a beamforming error and a grating lobe by increasing the resolution of a focal point formed by an ultrasound wave.

**Claims**

1. A beamforming apparatus for forming a beam focus by generating ultrasound waves at multiple channels, the beamforming apparatus comprising:

   a frequency selector configured to quasi-randomly select a driving frequency for each channel from two or more candidate frequencies;
   a phase selector configured to set a phase for each channel based on a wavelength of the driving frequency for each channel and a distance between a physical position of each channel and a focal point; and
   a beamforming signal generator configured to control generation of the ultrasound waves at the multiple channels, according to the driving frequency and the phase selected for each channel.

2. The beamforming apparatus of claim 1, wherein the candidate frequencies are frequencies exhibiting a response characteristic of 3 dB or more.

3. The beamforming apparatus of claim 1, wherein the driving frequency for each channel is selected randomly and differently from that of an adjacent channel.

4. The beamforming apparatus of claim 1, wherein the driving frequency for each channel is randomly selected so that selected driving frequencies have a uniform distribution.

5. The beamforming apparatus of claim 1, wherein the driving frequency for each channel is randomly selected so that the driving frequency for each channel is different from that of an adjacent channel and selected driving frequencies have uniform distribution.

6. The beamforming apparatus of claim 1, further comprising an amplitude selector configured to set an amplitude for each channel,
   wherein the beamforming signal generator generates the ultrasound waves according to the driving frequency, the phase and the amplitude selected for each channel.

7. The beamforming apparatus of claim 6, wherein the amplitude selector is configured to set the amplitude for each channel differently according to a distance from a center of a transducer of the beamforming apparatus to each channel.

8. The beamforming apparatus of claim 6, wherein the amplitude for each channel is selected randomly and differently from that of an adjacent channel.

9. The beamforming apparatus of claim 8, wherein the amplitude for each channel is set such that amplitudes are distributed by equal ratio with respect to individual channels.

10. A beamforming method performed by a beamforming apparatus for forming a beam focus by generating ultrasound waves at multiple channels, the beamforming method comprising:

    quasi-randomly selecting a driving frequency for each channel from two or more candidate frequencies;
    setting a phase for each channel based on a wavelength of the driving frequency for each channel and a distance between a physical position of each channel and a focal point; and

generating the ultrasound waves at the multiple channels, according to the driving frequency and the phase selected for each channel.

11. The beamforming method of claim 10, wherein the driving frequency for each channel is selected randomly and differently from that of an adjacent channel.

12. The beamforming method of claim 10, wherein the driving frequency for each channel is randomly selected so that the driving frequency for each channel is different from that of an adjacent channel and selected driving frequencies have uniform distribution.

13. The beamforming method of claim 10, further comprising differently setting an amplitude for each channel according to a distance from a center of a transducer of the beamforming apparatus to each channel,

wherein the ultrasound waves are generated according to the driving frequency, the phase and the amplitude selected for each channel.

**FIG. 1**

<u>200</u>

*210*

```
Frequency
Selector
```

*220*                                    *240*

```
Phase                Beamforming
Selector             Signal Generator
```

*230*

```
Amplitude
Selector
```

# FIG. 2

EP 2 799 112 A1

(a) Rectangular Array     (b) Annular Array

(c) Oval Array     (d) Random Array

*FIG. 3*

10

**FIG. 4**

**FIG. 5**

*FIG. 6*

**FIG. 7**

**Intensity Level along the Depth**

FIG. 8

Start

Randomly Select Driving Frequency for Each
Ultrasound Beam of Each Channel from Two or
More Candidate Frequencies    —S910

Set Phase for Each Channel Based on Wavelength of
Selected Driving Frequency for Each Channel and
Distance between Physical Location of Each
Channel and Focal Point    —S920

Set Different Amplitude According to Distance
between Center of Transducer and Channel    —S930

Generate Ultrasound Waves at Multiple Channels,
According to Driving Frequency and Phase Selected
for Each Channel or Generate Ultrasound Waves
According to Driving Frequency, Phase and
Amplitude Selected for Each Channel    —S940

End

*FIG. 9*

<div align="center">

**INTERNATIONAL SEARCH REPORT**

</div>

| International application No. |
|---|
| **PCT/KR2011/010338** |

| A. CLASSIFICATION OF SUBJECT MATTER |
|---|
| *A61N 7/00(2006.01)i, A61B 18/04(2006.01)i, A61B 8/08(2006.01)i* |
| According to International Patent Classification (IPC) or to both national classification and IPC |

| B. FIELDS SEARCHED |
|---|
| Minimum documentation searched (classification system followed by classification symbols) |
| A61N 7/00; A61B 17/22; A61B 8/00; A61N 7/02; A61B 18/18 |
| Documentation searched other than minimum documentation to the extent that such documents are included in the fields searched<br>Korean Utility models and applications for Utility models: IPC as above<br>Japanese Utility models and applications for Utility models: IPC as above |
| Electronic data base consulted during the international search (name of data base and, where practicable, search terms used)<br>eKOMPASS (KIPO internal) & Keywords: ultrasonic waves, beamforming device, focus, resolution, frequency, phase, random, select |

| C. DOCUMENTS CONSIDERED TO BE RELEVANT | | |
|---|---|---|
| Category* | Citation of document, with indication, where appropriate, of the relevant passages | Relevant to claim No. |
| A | WO 2011-080631 A2 (KONINKLIJKE PHILIPS ELECTRONICS, N.V.) 07 July 2011<br>See page 5, line 23 - page 6, line 5, page 15, line 5 - page 16, line 2, claims 2-4, 11-12, 16 and 18-19 and figures 2-3, 5 and 9. | 1-13 |
| A | US 6,007,499 A (MARTIN, ROY W. et al.) 28 December 1999<br>See column 12, line 54 - column 13, line 29, column 14, lines 29-32, column 16, lines 5-28 and figures 8-12. | 1-13 |
| A | US 2008-0015435 A1 (CRIBBS, ROBERT et al.) 17 January 2008<br>See paragraphs [0072]-[0076], [0092]-[0094] and [0105]-[0107], claim 2 and figures 5A-10 and 14. | 1-13 |
| A | WO 2010-006293 A2 (CORNELL UNIVERSITY) 14 January 2010<br>See paragraphs [0045] and [0088]-[0089], claims 16-19 and figures 17-18. | 1-13 |
| A | US 6,042,556 A (BEACH, KIRK W. et al.) 28 March 2000<br>See column 6, lines 44-45 and 55-60, column 9, line 66 - column 10, line 3, column 10, lines 56-58, claims 1, 7 and 13 and figures 2-3 and 6-8. | 1-13 |

☐ Further documents are listed in the continuation of Box C.  ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 24 SEPTEMBER 2012 (24.09.2012) | **24 SEPTEMBER 2012 (24.09.2012)** |
| Name and mailing address of the ISA/KR<br>Korean Intellectual Property Office<br>Government Complex-Daejeon, 189 Seonsa-ro, Daejeon 302-701, Republic of Korea<br>Facsimile No. 82-42-472-7140 | Authorized officer<br><br>Telephone No. |

Form PCT/ISA/210 (second sheet) (July 2009)

INTERNATIONAL SEARCH REPORT
Information on patent family members

International application No.

**PCT/KR2011/010338**

| Patent document cited in search report | Publication date | Patent family member | Publication date |
|---|---|---|---|
| WO 2011-080631 A2 | 07.07.2011 | WO 2011-080631 A3 | 10.11.2011 |
| US 6,007,499 A | 28.12.1999 | AU 1999-12049 A1 | 24.05.1999 |
| | | EP 1028660 A1 | 23.08.2000 |
| | | EP 1028660 B1 | 16.01.2008 |
| | | US 2003-0018255 A1 | 23.01.2003 |
| | | US 2007-0004984 A1 | 04.01.2007 |
| | | US 6315741 B1 | 13.11.2001 |
| | | US 6432067 B1 | 13.08.2002 |
| | | WO 99-22652 A1 | 14.05.1999 |
| US 2008-0015435 A1 | 17.01.2008 | AU 2003-219843 A1 | 09.09.2003 |
| | | AU 2003-219843 B2 | 23.04.2009 |
| | | CA 2476873 A1 | 28.08.2003 |
| | | EP 1476080 A1 | 17.11.2004 |
| | | JP 2005-517488 A | 16.06.2005 |
| | | JP 4551090 B2 | 22.09.2010 |
| | | US 2004-0039312 A1 | 26.02.2004 |
| | | US 7258674 B2 | 21.08.2007 |
| | | US 7841984 B2 | 30.11.2010 |
| | | WO 03-070105 A1 | 28.08.2003 |
| WO 2010-006293 A2 | 14.01.2010 | CN 102149429 A | 10.08.2011 |
| | | EP 2310093 A2 | 20.04.2011 |
| | | US 2011-285244 A1 | 24.11.2011 |
| | | WO 2010-006293 A3 | 14.05.2010 |
| | | WO 2010-006293 A9 | 18.11.2010 |
| US 6,042,556 A | 28.03.2000 | AU 1999-61330 A1 | 27.03.2000 |
| | | WO 00-13598 A1 | 16.03.2000 |

Form PCT/ISA/210 (patent family annex) (July 2009)